# EUROPÄISCHE PATENTSCHRIFT

(11) **EP 1 939 171 B1**
(45) Veröffentlichungstag und Bekanntmachung des Hinweises auf die Patenterteilung: **03.11.2010**
(21) Anmeldenummer: 07024257.3
(22) Anmeldetag: 14.12.2007
(51) Int. Cl.: C07C 263/20

(54) **Verfahren zur Konditionierung von Isocyanaten**
Method for conditioning isocyanates
Procédé destiné au conditionnement d'isocyanates

(30) Priorität: 27.12.2006 DE 102006061536
(43) Veröffentlichungstag der Anmeldung: 02.07.2008
(73) Patentinhaber: Bayer MaterialScience AG, 51368 Leverkusen (DE)
(72) Erfinder: Richter, Frank, 51373 Leverkusen (DE); Hecking, Andreas, 40764 Langenfeld (DE); Brahm, Martin, 51519 Odenthal (DE)

(56) Entgegenhaltungen:
- EP-A- 0 508 216
- EP-A- 1 053 998

## Beschreibung

Die Erfindung betrifft ein neues Verfahren zur Konditionierung von Isocyanaten.

Bei der Herstellung und Verarbeitung von Isocyanaten werden zuweilen phosphororganische Hilfsmittel und Zuschlagstoffe verwendet, die als solche oder in Form von Folgeprodukten zu Problemen bei der späteren Verarbeitung der Isocyanate führen können.

So finden sich beispielsweise in den nach der Lehre der DE-A 1670720 zugänglichen, Uretdiongruppen enthaltenen Polyisocyanaten zuweilen die Oxide der als Katalysator eingesetzten Trialkylphosphane, die mitunter aus anderen P-haltigen Zwischenprodukten, insbesondere sind das Trialkyldihalogenphosphorane, hervorgehen können. All diese P-haltigen Spezies können sich bei dem wiederholten Einsatz des im Verfahren typischerweise durch Destillation abgetrennten Monomers im Prozess anreichern und zu Problemen beim erneuten Einsatz des monomeren Isocyanates führen. Eine einfache destillative Abtrennung scheitert an der katalytischen Aktivität insbesondere der Trialkylphosphanoxide gegenüber Isocyanaten, die dazu führt, dass man unakzeptabel hohe Ausbeuteverluste in Kauf nehmen müsste.

Das Dokument EP-A 0508216 beschreibt ein Verfahren zur Konditionierung und/oder Reinigung von Isocyanaten bei welchem man das Isocyanat bei 20 bis 150°C mit 0,001 bis 1 Mol-%, bezogen auf die Menge des Isocyanates, einer silylierten Säure vermischt und gegebenenfalls die so erhaltene Mischung nach einer Verweildauer von mindestens 5 Minuten einer destillativen Aufarbeitung unterzieht.

Es hat nicht an Versuchen gefehlt, die störenden P-organischen Komponenten auf geeignete Weise zu entfernen (Konditionierung), wobei sich jedoch herausstellte, dass entweder keine Veränderung dieser störenden P-organischen Komponenten eintrat, oder aber dass das zu konditionierende Isocyanat in unakzeptablem Ausmaß mit dem Konditionierungsmittel reagierte.

Die Überführung von phosphororganischen Verbindungen o.g. Typs in Phosphansulfide mittels P₄S₁₀ ist zumindest für einige solcher Verbindungen beispielsweise in Houben-Weil, Bd. XII/1, S. 168 ff und darin zitierter Literatur beschrieben. Die Eignung zur Umwandlung von Triaklyphosphanoxiden in die entsprechenden Triaklyphosphansulfide mittels P₄S₁₀ gelingt nach Houben-Weil, Bd. E2 (Organische Phosphorverbindungen II), Seite 87 ff und darin zitierter Literatur allerdings nur in Einzelfällen. Ob und in wie fern diese Umwandlungsprodukte gegenüber NCO-Gruppen inert sind und eine Eignung zur Umwandlung und Abtrennung von Triaklyphosphanoxiden aus Isocyanaten vermutet werden könnte, ist dem Stand der Technik nicht zu entnehmen.

Es bestand nun die Aufgabe, ein geeignetes Verfahren zur Beseitigung von Trialkylphosphanoxiden und Trialkyldihalogenphosphoranen aus Isocyanaten oder deren Mischungen bereitzustellen, das es gestattet, die zu konditionierenden Isocyanate in hoher Ausbeute und Reinheit zurück zu gewinnen, ohne störende Nebenreaktionen mit dem Konditioniermittel oder seinen Folgeprodukten befürchten zu müssen.

Überraschenderweise wurde nun gefunden, dass die Behandlung von Trialkylphosphanoxidhaltigen Isocyanaten und -mischungen, die ggf. mit weiteren P-haltigen Verunreinigungen wie z.B. Trialkyldihalogenphosphoranen kontaminiert sein können, mit P₄S₁₀ als Konditioniermittel die vorstehende Aufgabe löst.

Gegenstand der vorliegenden Erfindung ist daher ein Verfahren zur zumindest teilweisen Beseitigung von Trialkylphosphanoxiden und ggf. auch Trialkyldihalogenphosphoranen aus Isocyanaten oder Isocyanatmischungen, bei dem die vorstehend genannten Phosphorverbindungen mit P₄S₁₀ unter zumindest anteiliger Bildung von Phosphansulfiden umgesetzt werden.

Die enthaltenden Trialkylphosphanoxide bzw. Trialkyldihalogenphosphorane entsprechen bevorzugt der allgemeinen Formel (I):

R,R',R"PX,Y (I),

wobei
R, R' und R" für gleiche oder verschiedene, organische Gruppen mit 1 bis 20 C Atomen, die in der Kette auch Heteroatome wie Sauerstoff, Stickstoff oder Schwefel tragen können, und
X,Y im Fall der Trialkylphosphanoxide gemeinsam für ein Sauerstoffatom oder im Fall der Trialkyldihalogenphosphorane für zwei, gleiche oder verschiedene Halogenatome wie Fluor, Chlor, Brom oder Iod stehen.

Die zugrunde liegenden Isocyanate oder Mischungen können an sich alle dem Fachmann bekannten NCO-gruppenhaltigen Verbindungen, einschließlich der an sich bekannten höhermolekularen Isocyanat-Folgeprodukte mit Urethan-, Allophanat-, Harnstoff, Biuret-, Uretdion-, Carbodiimid-, Uretonimin-, Isocyanurat- und/oder Iminooxadiazindionstruktur sein.

Bevorzugt sind monomere, niedermolekulare Isocyanate mit M_{w} ≤ 500 wie Toluylendiisocyanat (TDI), Naphtylendiisocyanat (Desmodur^{®} 15, Bayer MaterialScience AG, Leverkusen, DE), 4(2)isocyanatophenyl-methyl-4-isocyanatobenzol (4,4'- bzw. 2,4'-MDI), Hexamethylendiisocyanat (HDI), Methylpentandiisocyanat (MPDI), Trimethylhexandiisocyanat (TMDI), Bis(isocyanatomethyl)cyclohexan (H₆XDI), Norbornandiisocyanat (NBDI), Isophorondiisocyanat, Bis(isocyanatocyclohexyl)methan (H₁₂MDI) sowie deren beliebige Mischungen.

Die Menge des im erfindungsgemäßen Verfahren einzusetzenden P₄S₁₀ richtet sich in erster Linie nach der Art und dem Grad der phosphorhaltigen Verunreinigungen des Isocyanates. Eine solche Analyse ist für den Fachmann im Rahmen von Routineanalysen mittels Elementaranalyse und NMR-Spektroskopie problemlos möglich.

P₄S₁₀ wird dabei im molaren Verhältnis, bezogen auf den Phosphorgehalt des zu konditionierenden Isocyanates, 0,1 : 1 bis 1 : 1, bevorzugt 0,2 : 1 bis 0,5 : 1 eingesetzt.

Bevorzugt wird das erfindungsgemäße Verfahren solange geführt, bis der Anteil der P-haltigen Verunreinigungen, bezogen auf die ursprünglich enthaltende Menge, weniger als 20%, bevorzugt weniger als 10% beträgt.

Das erfindungsgemäße Verfahren wird im Temperaturbereich 0°C bis 150°C, bevorzugt 40°C bis 120°C durchgeführt.

Im Anschluss an die Umsetzung mit P₄S₁₀ kann die Reaktionsmischung einschließlich ggf. enthaltendem Lösemittel mit Hilfe der an sich bekannten Separationstechniken wie Destillation, Extraktion oder Kristallisation/Filtration aufgereinigt werden. Stört die Gegenwart des Konditioniermittel und seiner Folgeprodukte nicht, kann das konditionierte Isocyanat(gemisch) auch ohne weitere Aufarbeitung verwendet werden.

Bevorzugt ist zunächst eine Feststoffseparation zur Abtrennung von nicht umgesetztem P₄S₁₀ sowie der unlöslichen Umwandlungsprodukte aus der Reaktion von P₄S₁₀ mit den störenden P-haltigen Verunreinigungen.. Anschließend erfolgt eine Destillation, ggf. in der speziellen Ausführungsform der Dünnschichtdestillation. Selbstverständlich können auch Kombinationen zweier oder mehrerer dieser Techniken angewendet werden.

Es ist für die Durchführung des erfindungsgemäßen Verfahrens unerheblich, ob das Verfahren ganz oder teilweise diskontinuierlich oder kontinuierlich durchgeführt wird.

Weiterhin können im erfindungsgemäßen Verfahren zu einem beliebigen Zeitpunkt, in der Polyisocyanatchemie übliche Additive und Stabilisatoren zugesetzt werden. Beispiele sind Antioxidanzien, wie z.B. sterisch gehinderte Phenole (2,6-Di-tert.butylphenol, 4-Methyl-2,6-di-tert.butylphenol), Lichtschutzmittel, wie z.B. HALS-Amine, Triazole etc..

### Beispiele

Alle Angaben zu Mengenanteilen (%, ppm) beziehen sich, so nicht anders vermerkt, auf die Masse.

Die Ermittlung des NCO-Gehaltes erfolgt durch Titration gemäß DIN 53 185.

Die Angabe mol-% bzw. des molaren Verhältnisses unterschiedlicher Strukturtypen zueinander basiert auf ³¹P-NMR-spektroskopischen Messungen.

Der Phosphorgehalt aller Proben wurde durch Röntgenfluoreszenzanalyse (RFA) bestimmt.

Der überwiegende Teil der Reaktionen wird beispielhaft mit HDI als zu konditionierendem Isocyanat beschrieben. Das geschieht nur zur Verdeutlichung der Vorteile des erfindungsgemäßen Verfahrens und soll keine Einschränkung der vorliegenden Erfindung auf die beschriebenen Systeme bzw. Reaktionsbedingungen bedeuten.

P₄S₁₀ wurde von der Firma Aldrich, 82018 Taufkirchen, DE kommerziell erworben und ohne weitere Reinigung eingesetzt.

### Beispiel 1 (erfindungsgemäß)

Zu 1000 g Hexamethylendiisocyanat (HDI) das 360 ppm Phosphor enthielt, über 95 mol-% davon in der Form des Tributylphosphanoxides, wurden 3 g P₄S₁₀ in fester Form gegeben und anschließend 48 h bei Zimmertemperatur gerührt. Ein Teil des P₄S₁₀ ging dabei in Lösung. Anschließend wurde filtriert und das Filtrat mittels Dünnschichtdestillation bei 0,1 mbar aufgearbeitet, wobei Verdampfertemperatur und Dosierrate so gewählt wurden, dass ca. 10 % Schwersiederablauf und ca. 90 % farbloses Destillat anfielen. Das Destillat enthielt 180 ppm Phosphor, der zu über 95 mol-% in der Form des Tributylphosphansulfides vorlag.

### Beispiel 2 (erfindungsgemäß)

Zu 1000 g HDI das 580 ppm Phosphor enthielt, ca. 50 mol% davon in der Form des Tributylphosphanoxides und ca. 50 mol-% in der Form des Tributyldifluorphosphorans, wurden 3,3 g P₄S₁₀ in fester Form gegeben und anschließend 48 h bei Zimmertemperatur gerührt. Ein Teil des P₄S₁₀ ging dabei in Lösung. Anschließend wurde filtriert und das Filtrat mittels Dünnschichtdestillation bei 0,2 mbar aufgearbeitet, wobei Verdampfertemperatur und Dosierrate so gewählt wurden, dass ca. 5 % Schwersiederablauf und ca. 95 % farbloses Destillat anfielen. Das Destillat enthielt 210 ppm Phosphor, der zu über 95 mol-% in der Form des Tributylphosphansulfides vorlag. Das Signal des Tributylphosphanoxides trat im ³¹P-NMR Spektrum nicht mehr auf, das Triplett des Tributyldifluorphosphorans war nur noch äußerst intensitätsschwach zu erkennen.

Die in den Versuchen 1 und 2 erhaltenen Destillationsrückstände (Schwersiederablauf) waren bei Zimmertemperatur hochviskos, ließen sich aber durch Temperaturerhöhung mühelos wieder aufschmelzen und konnten, nach Additivierung mit weiterem P₄S₁₀, erneut zur Konditionierung eingesetzt werden.

### Beispiel 3 (erfindungsgemäß)

Eine mit P₄S₁₀ konditionierte Produktmischung, erhalten wie in Beispiel 2 beschrieben, wurde an Stelle der Dünnschichtdestillation einer Blasendestillation (Rektifikation) an einer gepackten Kolonne (evakuierter, innen verspiegelter Doppelmantel, 50 cm lang, Durchmesser 40 mm, gefüllt mit 4 mm V4A-Drahtwendeln) unterworfen, wobei über Kopf 99,96 % (GC) reines HDI (96,4 % der Gesamtmenge) mit einem Phosphorgehalt < 10 ppm übergingen. Der Habitus des Destillationsrückstandes war völlig analog dem unter 1 und 2 beschriebenen.

### Beispiel 4 (Vergleichsbeispiel)

Das kontaminierte HDI aus Beispiel 2 wurde ohne vorherige Behandlung mit P₄S₁₀ einer Blasendestillation (Rektifikation) analog Beispiel 3 unterworfen, wobei nach Abnahme eines Vorlaufs (Kp bis 74 °C bei 0,2 mbar Kopfdruck, 7,5 % der Gesamtmenge, ca. 99,0 % reines HDI) als Hauptlauf (59,6 % der Gesamtmenge) 99,9 % reines HDI mit Kp 74+/-1 °C bei 0,2 mbar Kopfdruck und einem Phosphorgehalt < 10 ppm übergingen. Der im Verlaufe der Destillation immer viskoser werdende Sumpf (32,9 % der Gesamtmenge) erstarrte nach dem Erkalten zu einer gallertartigen Masse, die sich nicht wieder aufschmelzen ließ, in organischen Lösungsmitteln nur teilweise löslich war und nur durch Zerschlagen des Glaskolbens isoliert werden konnte. Analytisch fanden sich neben Hinweisen auf das Vorliegen freier NCO-Gruppen hauptsächlich Belege für die Bildung von Carbodiimiden und Uretoniminen. Der Phosphorgehalt des Sumpfproduktes betrug 110 ppm.

## Patentansprüche

1. Verfahren zur zumindest teilweisen Beseitigung von Trialkylphosphanoxiden und Trialkyldihalogenphosphoranen aus Isocyanaten oder Isocyanatmischungen, **dadurch gekennzeichnet, dass** die Trialkylphosphanoxide und Trialkyldihalogenphosphorane mit P₄S₁₀ unter zumindest anteiliger Bildung von Trialkylphosphansulfiden umgesetzt werden.

2. Verfahren gemäß Anspruch 1, **dadurch gekennzeichnet, dass** die Trialkylphosphanoxide und Trialkyldihalogenphosphorane der allgemeinen Formel (I) entsprechen
R,R',R"PX,Y (I),
wobei
R, R' und R" für gleiche oder verschiedene, organische Gruppen mit 1 bis 20 C Atomen, die in der Kette auch Heteroatome wie Sauerstoff, Stickstoff oder Schwefel tragen können, und
X,Y im Fall der Trialkylphosphanoxide gemeinsam für ein Sauerstoffatom oder im Fall der Trialkyldihalogenphosphorane für zwei, gleiche oder verschiedene, Halogenatome wie Fluor, Chlor, Brom oder Iod stehen.

3. Verfahren gemäß Anspruch 1 oder 2, **dadurch gekennzeichnet, dass** das molare Verhältnis von P₄S₁₀ bezogen auf den Phosphor der im zu konditionierende Isocyanat vorliegenden phosphorhaltigen Verbindungen von 0,1 : 1 bis 1 : 1 beträgt.

4. Verfahren gemäß einem der Ansprüche 1 bis 3, **dadurch gekennzeichnet, dass** das Verfahren solange geführt, bis der Anteil der Trialkylphosphanoxide oder der Trialkyldihalogenphosphorane bezogen auf die ursprünglich enthaltende Menge weniger als 10 % beträgt.

5. Verfahren gemäß einem der Ansprüche 1 bis 4, **dadurch gekennzeichnet, dass** im Anschluss an die Reaktion zunächst eine Feststoffseparation zur Abtrennung von nicht umgesetztem P₄S₁₀ sowie der unlöslichen Umwandlungsprodukte der Reaktion von P₄S₁₀ mit den phosphorhaltigen Verunreinigungen durchgeführt wird.

6. Verfahren gemäß Anspruch 5, **dadurch gekennzeichnet, dass** anschließend an die Feststoffseparation eine Destillation erfolgt.

## Claims

1. Process for the at least partial elimination of trialkylphosphine oxides and also trialkyldihalophosphoranes from isocyanates or isocyanate mixtures, **characterized in that** the trialkylphosphine oxides and trialkyldihalophosphoranes are reacted with P₄S₁₀ with at least proportional formation of trialkylphosphine sulphides.

2. Process according to Claim 1, **characterized in that** the trialkylphosphine oxides and trialkyldihalophosphoranes correspond to the general formula (I)
R,R',R"PX,Y (I),
where
R, R' and R" are identical or different organic groups having 1 to 20 carbon atoms, which in the chain can also carry heteroatoms such as oxygen, nitrogen or sulphur,
and
X, Y in the case of trialkylphosphine oxides are together an oxygen atom, or in the case of trialkyldihalophosphoranes, are two, identical or different, halogen atoms such as fluorine, chlorine, bromine or iodine.

3. Process according to Claim 1 or 2, **characterized in that** the molar ratio of P₄S₁₀ based on the phosphorus to the phosphorus-containing compounds present in the isocyanate to be conditioned is 0.1:1 to 1:1.

4. Process according to one of Claims 1 to 3, **characterized in that** the process is carried out until the proportion of the trialkylphosphine oxides or of the trialkyldihalophosphoranes based on the amount originally present is less than 10%.

5. Process according to one of Claims 1 to 4, **characterized in that**, subsequently to the reaction, first a solids separation is carried out for separating off unreacted P₄S₁₀ and also the insoluble conversion products of the reaction of P₄S₁₀ with the phosphorus-containing impurities.

6. Process according to Claim 5, **characterized in that** a distillation is performed subsequently to the solids separation.

## Revendications

1. Procédé d'élimination au moins partielle d'oxydes de trialkylphosphane et de trialkyldihalogénophosphoranes d'isocyanates ou de mélanges d'isocyanates, **caractérisé en ce que** les oxydes de trialkylphosphane et les trialkyldihalogénophosphoranes sont mis en réaction avec P₄S₁₀, avec formation au moins proportionnelle de sulfures de trialkylphosphane.

2. Procédé selon la revendication 1, **caractérisé en ce que** les oxydes de trialkylphosphane et les trialkyldihalogénophosphoranes correspondent à la formule générale (I)
R,R',R"PX,Y (I),
dans laquelle
R, R' et R" représentent des groupes organiques identiques ou différents, contenant 1 à 20 atomes C, qui peuvent également porter des hétéroatomes tels que l'oxygène, l'azote ou le soufre dans la chaîne, et
X, Y représentent, dans le cas des oxydes de trialkylphosphane, collectivement un atome d'oxygène ou, dans le cas des trialkyldihalogénophosphoranes, deux atomes d'halogène, identiques ou différents, tels que le fluor, le chlore, le brome ou l'iode.

3. Procédé selon la revendication 1 ou 2, **caractérisé en ce que** le rapport molaire entre P₄S₁₀ et le phosphore des composés contenant du phosphore présents dans l'isocyanate à conditionner est de 0,1:1 à 1:1.

4. Procédé selon l'une quelconque des revendications 1 à 3, **caractérisé en ce que** le procédé est réalisé jusqu'à ce que la proportion des oxydes de trialkylphosphane ou des trialkyldihalogénophosphoranes soit inférieure à 10 % de la quantité initialement contenue.

5. Procédé selon l'une quelconque des revendications 1 à 4, **caractérisé en ce qu'**une séparation des solides pour la séparation du P₄S₁₀ non réagi et des produits de transformation insolubles de la réaction de P₄S₁₀ avec les impuretés contenant du phosphore est tout d'abord réalisée après la réaction.

6. Procédé selon la revendication 5, **caractérisé en ce qu'**une distillation a lieu après la séparation des solides.
